# EUROPEAN PATENT SPECIFICATION

(11) **EP 2 465 554 B1**
(45) Date of publication and mention of the grant of the patent: **20.08.2014**
(21) Application number: 12159657.1
(22) Date of filing: 05.10.2010
(51) Int. Cl.: A61M 1/32

(54) **Oxygenator with integrated arterial filter including filter frame**
Oxygenator mit einem integrierten arteriellen Filter aufweisend einen Filterrahmen
Oxygenateur avec un filtre artériel integré comprenant un cadre de filtre

(30) Priority: 29.04.2010 EP 10161451
(43) Date of publication of application: 20.06.2012
(62) Divisional of application: 10186550.9
(73) Proprietor: Sorin Group Italia S.r.l., 41037 Mirandola (MO) (IT)
(72) Inventor: Reggiani, Stefano, 41936 Medolla (MO) (IT); Giri, Alberto, 41037 Mirandola (MO) (IT); Silvestri, Claudio, 41037 Quarantoli Mirandola (Modena) (IT); Redaelli, Alberto, 20133 Milano (IT); Fiore, Gianfranco Beniamino, 20129 Milano (IT)
(74) Representative: Bosotti, Luciano

(56) References cited:
- EP-A1- 0 312 125
- WO-A2-97/16213
- US-A1- 2004 175 292
- US-B1- 6 241 945

## Description

### RELATED APPLICATION

This application claims priority from European Patent application EP 10 161451 filed April 29, 2010.

### TECHNICAL FIELD

The disclosure pertains generally to arterial filters used in blood perfusion systems.

### BACKGROUND

Blood perfusion entails encouraging blood through the vessels of the body. For such purposes, blood perfusion systems typically entail the use of one or more pumps in an extracorporeal circuit that is interconnected with the vascular system of a patient. Cardiopulmonary bypass surgery typically requires a perfusion system that provides for the temporary cessation of the heart to create a still operating field by replacing the function of the heart and lungs. Such isolation allows for the surgical correction of vascular stenosis, valvular disorders, and congenital heart defects. In perfusion systems used for cardiopulmonary bypass surgery, an extracorporeal blood circuit is established that includes at least one pump and an oxygenation device to replace the functions of the heart and lungs.

More specifically, in cardiopulmonary bypass procedures oxygen-poor blood, i.e., venous blood, is gravity-drained or vacuum suctioned from a large vein entering the heart or other veins in the body (e.g., femoral) and is transferred through a venous line in the extracorporeal circuit. The venous blood is pumped to an oxygenator that provides for oxygen transfer to the blood. Oxygen may be introduced into the blood by transfer across a membrane or, less frequently, by bubbling oxygen through the blood. Concurrently, carbon dioxide is removed across the membrane. The oxygenated blood is filtered and then returned through an arterial line to the aorta, femoral, or other artery.

Often, an arterial filter is added to the extracorporeal circuit, after the oxygenator, as last barrier before the patient, so as to block any solid or gaseous emboli and prevent any such emboli from entering into the aorta of the patient. Recently, arterial filters integrated in the oxygenator have been developed, allowing the reduction of the priming volume of the circuit and decreasing the global haemodilution of the patient. Document US2004/175292 discloses a blood processing apparatus comprising a filter before the blood outlet opening supported by ribs.

### SUMMARY

The invention is defined by the appended claims. Example 1 is a blood processing apparatus that includes an apparatus housing having a blood inlet and a blood outlet, the blood inlet extends into an interior of the apparatus housing. A heat exchanger is disposed about the blood inlet and in fluid communication therewith. A gas exchanger is disposed about the heat exchanger and in fluid communication therewith. A filter housing is coupled about the apparatus housing and defining a filter volume between the apparatus housing and the filter housing, the filter volume is in fluid communication with the gas exchanger via one (not according to the claims) or more (according to the claims) openings that are formed within the apparatus housing such that blood exiting the gas exchanger can pass into the filter volume. A filter assembly is disposed within the filter housing. The filter assembly includes a filter frame having one or more ribs and a filter net disposed on the filter frame. The plurality of ribs align with at least a portion of the one or more openings so as to reduce blood velocity through at least a portion of the filter net.

In Example 2, the blood processing apparatus of Example 1 in which the one or more openings comprise a plurality of openings arranged along an arcuate path. The one or more ribs comprise arcuate ribs aligned with the arcuate path.

In Example 3, the blood processing apparatus of Example 1 in which the filter frame has a first annular frame ring having a first diameter, a second annular frame ring having a second diameter, greater than the first diameter, and one or more bridge elements extending between the first annular frame ring and the second annular frame ring.

In Example 4, the blood processing apparatus of Example 3 in which one or more ribs are disposed between the first annular frame ring and the second annular frame ring.

In Example 5, the blood processing apparatus of Example 1 in which the filter frame includes a plate portion arranged near the blood outlet to limit preferential blood flow through the filter assembly near the blood outlet.

In Example 6, the blood processing apparatus of Example 1 in which the filter assembly divides the filter volume into a first chamber between the filter assembly and the apparatus housing and a second chamber between the filter assembly and the filter housing.

In Example 7, the blood processing apparatus of Example 6, in which further comprises a first purge port in fluid communication with the first chamber and a second purge port in fluid communication with the second chamber.

In Example 8, the blood processing apparatus of Example 7 in which the filter housing has a frustoconical configuration having a smaller diameter at one end and a larger diameter at an opposing end. The second purge port is located near the larger diameter end of the filter housing.

In Example 9, the blood processing apparatus of Example 8 in which the first purge port is located near the smaller diameter end of the filter housing.

In Example 10, the blood processing apparatus of Example 7 in which bubbles within the blood can exit through the first purge port and/or the second purge port.

In Example 11, the blood processing apparatus of Example 1 in which the gas exchanger is configured to permit gas to flow therethrough in order to add oxygen and remove carbon dioxide from the blood passing through the gas exchanger.

In Example 12, the blood processing apparatus of Example 1 in which the filter assembly includes a biocompatible coating on the filter net.

In Example 13, the blood processing apparatus of Example 1 in which the filter net comprises a polyester filter net or a polypropylene filter net.

Example 14 is a blood processing apparatus that includes an apparatus housing having a blood inlet and a blood outlet, the blood inlet extending into an interior of the apparatus housing. A heat exchanger core extends coaxially within the apparatus housing and is axially aligned with the blood inlet. Heat exchanger hollow fibers are disposed about the heat exchanger core such that a heat exchanger fluid may flow through the heat exchanger hollow fibers and blood passing from the blood inlet may flow across the heat exchanger hollow fibers. A cylindrical shell extends coaxially about the heat exchanger core. The cylindrical shell includes an annular shell aperture that is disposed near an end of the cylindrical shell opposite to an end near the blood inlet. The annular shell aperture is configured to permit blood to pass to an exterior of the cylindrical shell. Gas exchanger hollow fibers are disposed about the cylindrical shell such that gases may flow through the gas exchanger hollow fibers and blood passing from the annular shell aperture may flow across the gas exchanger hollow fibers. A filter housing is coupled about the apparatus housing and defines a filter volume between the apparatus housing and the filter housing. The filter volume is in fluid communication with the gas exchanger via one or more openings that are formed within the apparatus housing along an arcuate path such that blood exiting the gas exchanger can pass into the filter volume. A filter assembly is disposed within the filter housing and divides the filter volume into a first chamber between the filter assembly and the apparatus housing and a second chamber between the filter assembly and the filter housing. The filter assembly includes a filter frame with one or more arcuate ribs and a filter net disposed on the filter frame. The one or more arcuate ribs are aligned with the one or more openings along the arcuate path in order to slow blood velocity through the filter net. The blood processing apparatus includes a first purge port that is in fluid communication with the first chamber and a second purge port that is in fluid communication with the second chamber.

In Example 15, the blood processing apparatus of Example 14 in which the filter assembly includes a biocompatible coating on the filter net.

In Example 16, the blood processing apparatus of Example 14 in which the filter net comprises a polyester filter net or a polypropylene filter net.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

Figure 1 is a schematic illustration of a blood processing apparatus including an integrated arterial filter in accordance with an embodiment of the invention.

Figure 2 is a cross-sectional illustration of the blood processing apparatus of Figure 1.

Figure 3 is an illustrative view of a filter deployed within the blood processing apparatus of Figure 1.

Figure 4 is an illustrative view of another filter deployed within the blood processing apparatus of Figure 1.

Figure 5 is a schematic illustration of a blood processing apparatus including an integrated arterial filter in accordance with an embodiment of the invention.

Figure 6 is a schematic cross-sectional illustration of the blood processing apparatus of Figure 5.

Figure 7 is an illustrative view of a filter deployed within the blood processing apparatus of Figure 5.

Figure 8 is a schematic illustration of a blood processing apparatus including an integrated arterial filter in accordance with an embodiment of the invention.

Figure 9 is a perspective illustration of a blood processing apparatus including an integrated arterial filter in accordance with an embodiment of the invention.

Figure 10 is a cross-sectional view of the blood processing apparatus of Figure 9.

Figure 11 is a perspective illustration of a portion of the blood processing apparatus of Figure 9.

Figure 12 is a perspective illustration of a portion of the blood processing apparatus of Figure 9.

Figures 13 and 14 are schematic illustrations of blood flow showing how blood flow velocity is reduced in the blood processing apparatus of Figure 9.

Figures 15 and 16 are graphical representations illustrating how backflow is reduced in the blood processing apparatus of Figure 9.

### DETAILED DESCRIPTION

The disclosure pertains to a blood processing apparatus that combines, in a single structure, a heat exchanger, a gas exchanger or oxygenator and an arterial filter. In some embodiments, the term oxygenator may be used to refer to a structure that combines a heat exchanger, a gas exchanger and an arterial filter in a unitary device. In some embodiments, an oxygenator may be used in an extracorporeal blood circuit. An extracorporeal blood circuit, such as may be used in a bypass procedure, may include several different elements such as a heart-lung machine, a blood reservoir, as well as an oxygenator. In some embodiments, by incorporating the arterial filter with the oxygenator, the tubing set used to create the extracorporeal blood circuit may be reduced in complexity or number of parts and thus may simplify the extracorporeal blood circuit. In some embodiments, this will reduce the priming volume of the extracorporeal blood circuit.

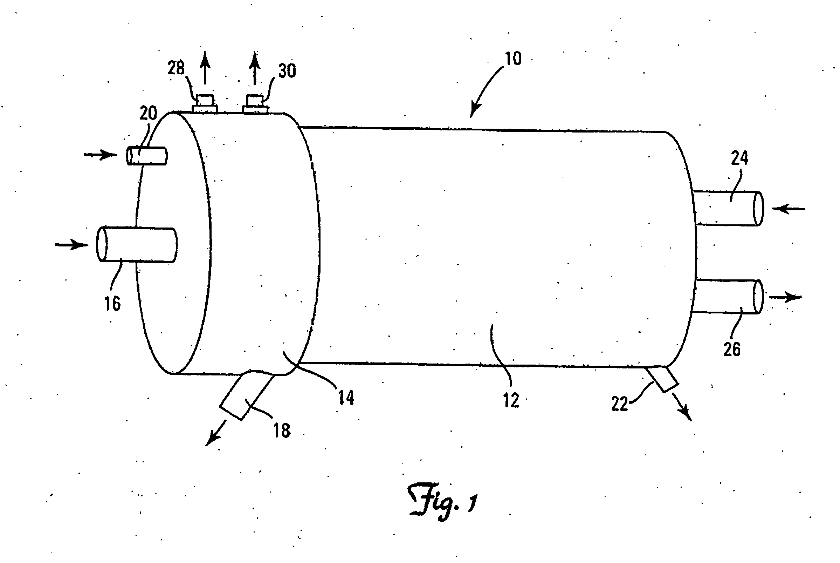

Figure 1 is a schematic illustration of a blood processing apparatus or oxygenator 10. While the internal components are not visible in this illustration, the oxygenator 10 may include one or more of a heat exchanger, a gas exchanger and an arterial filter. According to some embodiments, each of the heat exchanger, gas exchanger and arterial filter are integrated into a single structure that forms an oxygenator housing. The oxygenator 10 includes a device compartment or housing 12 and an arterial filter compartment or housing 14. In some embodiments, the arterial filter housing 14 may be integrally molded or otherwise structurally integrated with the device housing 12. In some cases, the arterial filter housing 14 may be separately formed and then secured or otherwise coupled to the device housing 12. According to various embodiments, the heat exchanger, the gas exchanger, and the arterial filter housing 14 may have a cross-section shaped generally as a circle or as a parallelogram (e.g., a square or rectangle). Each of the heat exchanger, the gas exchanger and the arterial filter housing 14 may have generally the same sectional shape or each may have a different sectional shape.

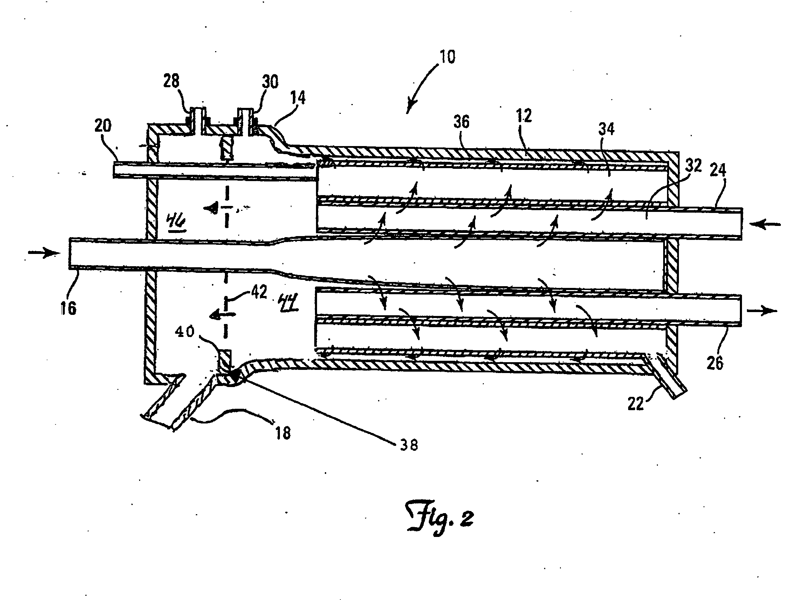

In some embodiments, a blood inlet 16 extends through the arterial filter housing 14 and into the device housing 12. A blood outlet 18 exits the arterial filter housing 14. As noted, in some embodiments the oxygenator 10 includes a gas exchanger and thus may include a gas inlet 20 and a gas outlet 22. In some embodiments, the oxygenator 10 includes a heat exchanger and thus may include a heating fluid inlet 24 and a heating fluid outlet 26. As will be explained in greater detail with respect to Figure 2, the oxygenator 10 includes a first purge port 30 and a second purge port 28. The positions of the inlets, outlets and purge ports are merely illustrative, as other arrangements and configurations are contemplated. The purge ports may include a valve or a threaded cap. The purge ports operate to permit gases (e.g., air bubbles) that exit the blood to be vented or aspirated and removed from the oxygenator.

Figure 2 is a cross-sectional view of the oxygenator 10, illustrating internal components and exemplary blood flow through the oxygenator 10. The oxygenator 10 includes a heat exchanger 32 and a gas exchanger 34. In some embodiments, the heat exchanger 32 includes a number of hollow fibers through which a heating fluid such as water can flow. The blood may flow around and past the hollow fibers and thus be suitably heated. In some embodiments, the hollow fibers may be polymeric. In some cases, metallic fibers may be used within the heat exchanger 32. According to other embodiments, the heat exchanger 32 includes a metal bellows or other structure comprising a substantial surface area (e.g., fins) for facilitating heat transfer with the blood.

In some embodiments the gas exchanger 34 may include a number of microporous hollow fibers through which a gas such as oxygen may flow. The blood may flow around and past the hollow fibers. Due to concentration gradients, oxygen may diffuse through the hollow fibers into the blood while carbon dioxide may diffuse into the hollow fibers and out of the blood.

The oxygenator 10, according to some embodiments, includes an annular space 36 into which blood may flow as the blood exits the gas exchanger 34. As illustrated, the annular space 36 may extend into the arterial filter housing 14. According to exemplary embodiments, the annular space 36 may be generally circular or generally rectangular. The arterial filter housing 14 includes a filter 38. In some embodiments, the filter 38 includes an annular frame 40 and a net or mesh 42 spanning the annular frame 40. In some embodiments, the filter 38 may be considered as dividing a volume within the arterial filter housing 14 into a first chamber 44 and a second chamber 46. In various embodiments, the annular frame 40 and the net or mesh 42 are disposed concentrically with respect to the filter housing 14. In other embodiments the annular frame 40 and the mesh 42 are disposed about the housing 14 in a non-concentric manner. According to exemplary embodiments, the internal (i.e., priming) volume of the arterial filter housing 14 is between about 30 to about 150 mL or from about 80 and about 110 mL. According to other embodiments, the priming volume is between about 30 to 150mL or from about 90 and about 100 mL.

In some embodiments, the annular space 36 may open into or otherwise be in fluid communication with the first chamber 44. While blood is in the first chamber 44, any air bubbles that are present within the blood may be vented through the first purge port 30. Blood may pass through the filter 38 and into the second chamber 46. Any bubbles remaining in the blood, or caused by passage through the filter 38, may be vented through the second purge port 28. Blood may then exit the oxygenator 10 through the blood outlet 18. The presence of the second purge port 28 in the second chamber 46 and the first purge port 30 in the first chamber 44, according to various embodiments, will improve the priming speed due to the fact that bubbles present in the blood have both a first and a second opportunity to exit through a purge port. Moreover, in these embodiments, the efficacy of the bubble or gas removal is improved, again due to the fact that bubbles present in the blood have both a first and a second opportunity to exit through a purge port.

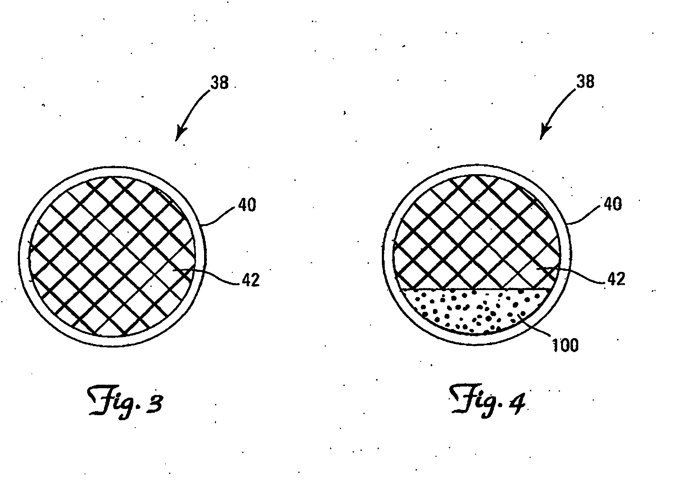

Figure 3 is a view of the filter 38, illustrating the frame 40 and the net or mesh 42. Figure 4 shows an embodiment of the filter 38 including a blocking plate 100. In some embodiments, the blocking plate 100 may be sized, shaped and positioned near the blood outlet 18 to limit preferential blood flow on the lower portion of the oxygenator 10. According to various embodiments, the filter 38 may have a cross-sectional shape that is circular, rectangular, or any other shape.

In some embodiments, the net or mesh 42 may have a mesh size that is the range of about 20 to about 200 microns. In some cases, the net or mesh 42 may have a mesh size of about 120 microns. In some instances, the net or mesh 42 may have a mesh size of from about 38-40 microns, and may be formed of a polymeric material such as polyester or polypropylene. In some cases, the net 42 may be coated with a biocompatible material. The blocking plate 100 may be formed of any suitable material. In some embodiments, the blocking plate 100 may be integrally formed with the frame 40. According to various exemplary embodiments, the net or mesh 42 has a surface area of between about 70 and about 90 square centimeters. According to other exemplary embodiments, the net or mesh 42 has a surface are of between about 75 and about 80 square centimeters.

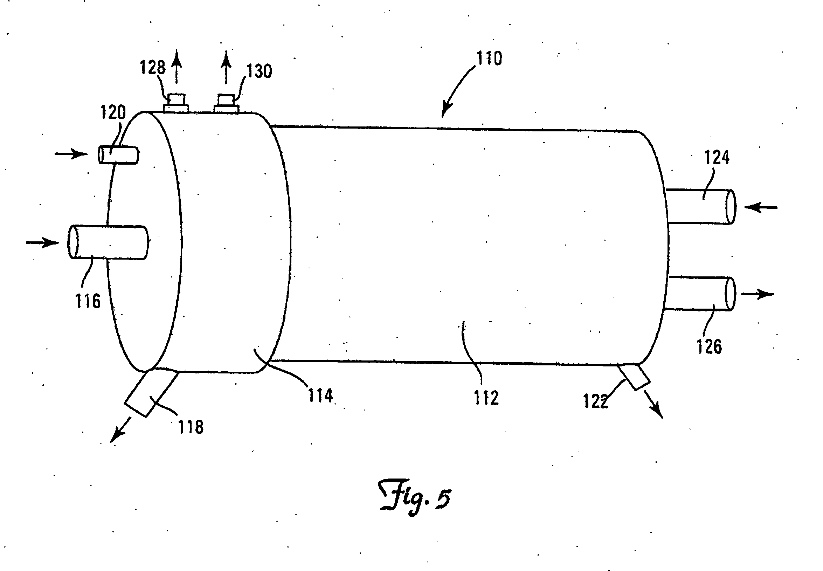

Figure 5 is a schematic illustration of a blood processing apparatus or oxygenator 110. While the internal components are not visible in this illustration, the oxygenator 110 may include one or more of a heat exchanger, a gas exchanger and an arterial filter. The oxygenator 110 includes a device housing 112 and an arterial filter housing 114. In some embodiments, the arterial filter housing 114 may be integrally molded or otherwise formed with the device housing 112. In some cases, the arterial filter housing 114 may be separately formed and then secured to the device housing 112.

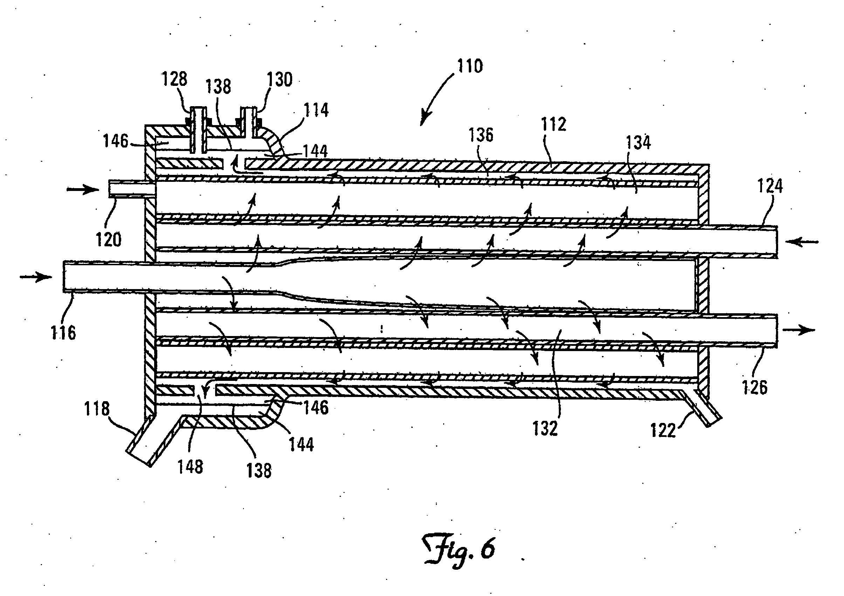

In some embodiments, a blood inlet 116 extends through the arterial filter housing 114 and into the device housing 112. A blood outlet 118 exits the arterial filter housing 114. As noted, in some embodiments the oxygenator 110 includes a gas exchanger and thus may include a gas inlet 120 and a gas outlet 122. In some embodiments, the oxygenator 110 includes a heat exchanger and thus may include a heating fluid inlet 124 and a heating fluid outlet 126. As will be explained in greater detail with respect to Figure 6, the oxygenator 110 includes a first purge port 128 and a second purge port 130. The positions of the inlets, outlets and purge ports are merely illustrative, as other arrangements and configurations are contemplated.

Figure 6 is a cross-sectional view of the oxygenator 110, illustrating internal components of the oxygenator 110. The oxygenator 110 includes a heat exchanger 132 and a gas exchanger 134. In some embodiments, the heat exchanger 132 includes a number of hollow polymeric or metallic fibers through which a heating fluid such as water can flow. The blood may flow around and past the hollow fibers and thus be suitably heated. In some embodiments the gas exchanger 134 may include a number of hollow fibers through which a gas such as oxygen may flow. The blood may flow around and past the hollow fibers. Due to concentration gradients, oxygen may diffuse through the hollow fibers into the blood while carbon dioxide may diffuse into the hollow fibers and out of the blood.

As shown in Figure 6, the gas exchanger is configured such that blood flows radially across the gas exchanger 134. In these embodiments, the oxygenator 110 includes an annular space 136 into which blood may flow as the blood exits the gas exchanger 134. According to various embodiments, the annular space 136 may be either open or it may be partially or completely filled with hollow fibers. As illustrated, the arterial filter housing 114 may extend over a portion of the annular space 136. According to other embodiments, the gas exchanger 134, the heat exchanger 132, or both may be configured such that blood is directed in a longitudinal flow path. In various exemplary embodiments where the gas exchanger 134 is configured such that blood flows in a longitudinal path, the annular space 136 is omitted. In these embodiments, the blood flows out of the gas exchanger 134 near an end and flows directly into the arterial filter housing 114. In some embodiments, the opening between the gas exchanger 134 and the arterial filter housing 114 is blocked or occluded at the radial location corresponding to the blood outlet 118 of the arterial filter housing 114, to minimize or prevent direct flow from the gas exchanger 134 into the blood outlet 118.

A filter 138 may be disposed within the arterial filter housing 114. In some instances, as illustrated, the filter 138 divides the space within the annular filter housing 114 into a first chamber 144 and a second chamber 146. An opening 148 that may extend circumferentially up to about 360 degrees provides fluid communication between the annular space 136 and the first chamber 144. While blood is in the first chamber 144, any air bubbles that are present within the blood may be vented through the first purge port 128. Blood may pass through the filter 138 and into the second chamber 146. Any bubbles remaining in the blood, or caused by passage through the filter 138, may be vented through the second purge port 130. Blood may then exit the oxygenator 110 through the blood outlet 118.

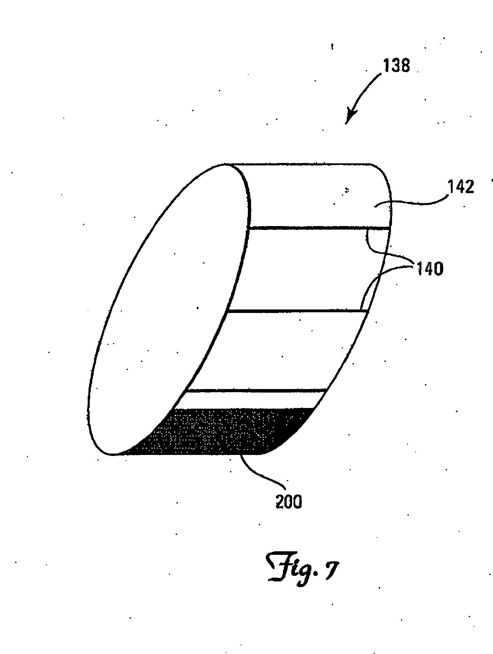

Figure 7 is a view of the filter 138. In some embodiments, the filter 138 is a cylindrical filter that includes one or more reinforcements 140 and a cylindrical net or mesh 142. In some embodiments, the one or more reinforcements 140 may be molded into the cylindrical net or mesh 142. In some cases, the one or more reinforcements 140 may be adhesively secured to the cylindrical net or mesh 142. In some embodiments, the one or more reinforcements 140 may extend cylindrically about the filter 138. In some instances, the one or more reinforcements 140 may run across the filter 138.

In some embodiments, the net or mesh 142 may have a mesh size that is the range of about 20 to about 200 microns. In some cases, the net or mesh 142 may have a mesh size of about 120 microns. In some instances, the net or mesh 142 may have a mesh size of about 40 microns, and may be formed of a polymeric material such as polyester or polypropylene. In some cases, the net 142 may be coated with a biocompatible material.

In some embodiments, the net or mesh 142 may include a blocking region or plate 200 that is sized, shaped and positioned near the blood outlet 118 to limit preferential blood flow on the lower portion of the oxygenator 110. The blocking plate 200 may be formed of any suitable material. In some embodiments, the blocking plate 200 may be molded or otherwise formed within the net or mesh 142.

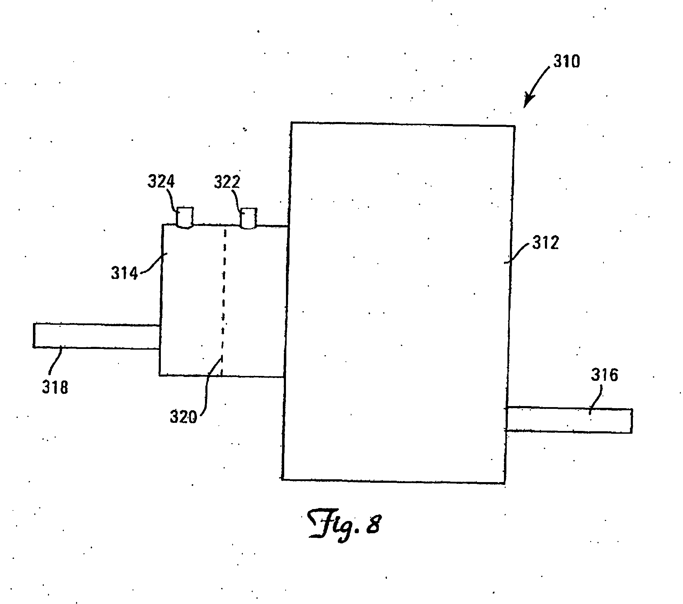

Figure 8 is a schematic illustration of a blood processing apparatus or oxygenator 310. While the internal components are not visible in this illustration, the oxygenator 310 may include one or more of a heat exchanger, a gas exchanger and an arterial filter. The oxygenator includes a device housing 312 and an arterial filter housing 314. In the illustrated embodiment, the arterial filter housing 314 is integrated into an end or side face of the device housing 312 and is configured such that blood exiting the device housing 312 enters the arterial filter housing 314. The device housing 312 includes a blood inlet 316 while the arterial filter housing 314 includes a blood outlet 318.

In some embodiments, as illustrated, the arterial filter housing 314 includes a net filter 320, a first purge port 322 and a second purge port 324. The first purge port 322 may be in fluid communication with an interior of the arterial filter housing 314 at a position upstream of the net filter 320 while the second purge port 324 may be in fluid communication with an interior of the arterial filter housing 314 at a position downstream of the net filter 320. As described in more detail above, this configuration allows an improvement and priming speed and efficacy, while also reducing the overall priming volume.

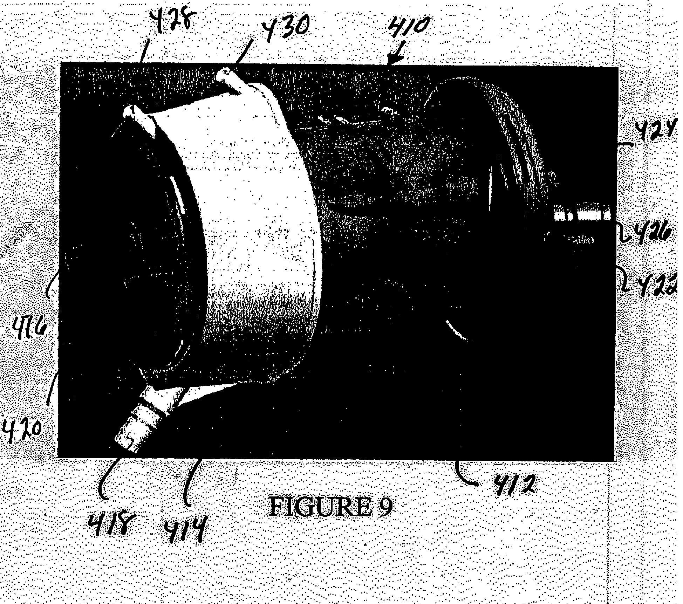

Figure 9 is a schematic illustration of a blood processing apparatus 410. While the internal components are not visible in this illustration, the blood processing apparatus 410 may include one or more of a heat exchanger, a gas exchanger and an arterial filter. According to some embodiments, each of the heat exchanger, gas exchanger and arterial filter are integrated into a single structure. The blood processing apparatus 410 includes an apparatus housing 412 and a filter housing 414. In some embodiments, the filter housing 414 may be integrally molded or otherwise structurally integrated with the apparatus housing 412. In some cases, the filter housing 414 may be separately formed and then secured or otherwise coupled to the apparatus housing 412. In some embodiments, as illustrated, the filter housing 414 may be considered as having a tapered or frustoconical shape.

In some embodiments, a blood inlet 416 extends through the filter housing 414 and into the apparatus housing 412. A blood outlet 418 exits the filter housing 414. As noted, in some embodiments the blood processing apparatus 410 includes a gas exchanger and thus may include a gas inlet 420 and a gas outlet 422. In some embodiments, the blood processing apparatus 410 includes a heat exchanger and thus may include a heating fluid inlet 424 and a heating fluid outlet 426. In some embodiments, the blood processing apparatus 410 may include a first purge port 428 and a second purge port 430. The positions of the inlets, outlets and purge ports are merely illustrative, as other arrangements and configurations are contemplated. The purge ports may include a valve or a threaded cap. The purge ports operate to permit gases (e.g., air bubbles) that exit the blood to be vented or aspirated and removed from the blood processing apparatus 410.

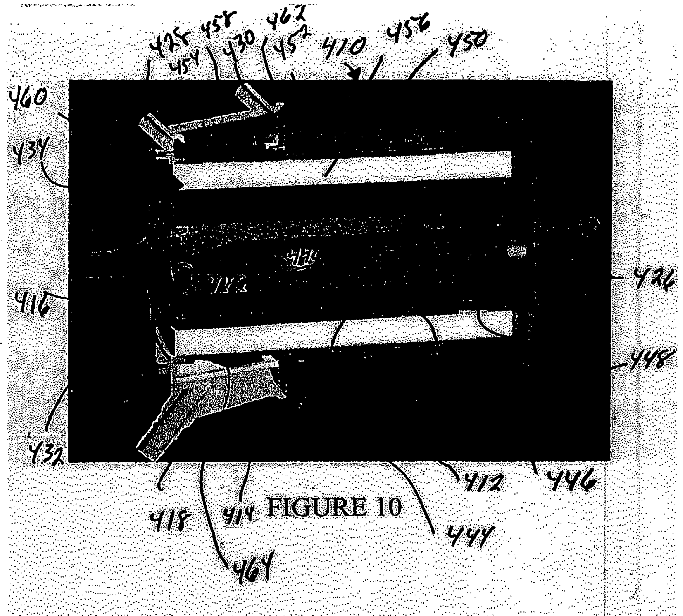

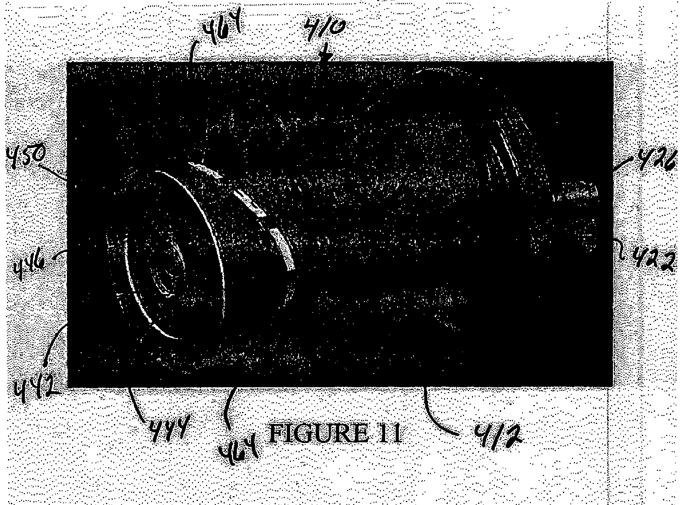

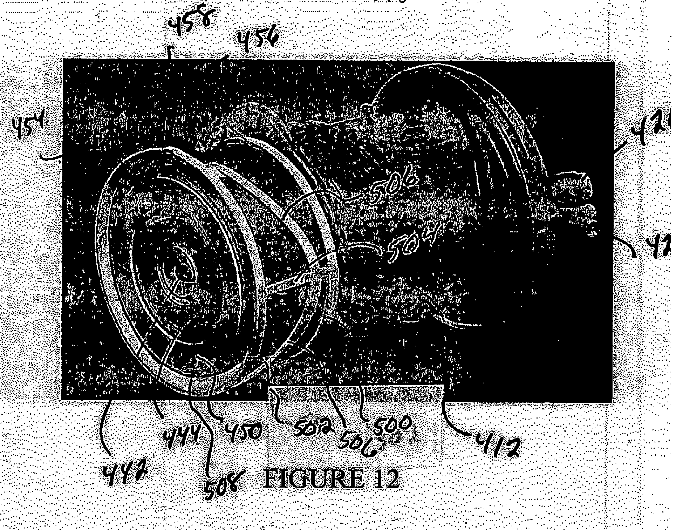

Figures 10, 11 and 12 further illustrate portions of the blood processing apparatus 410. Figure 10 is a cross-sectional view of the blood processing apparatus 410, while Figures 11 and 12 are perspective views with some elements or features removed to illustrate underlying structure.

The blood processing apparatus 410 includes a heat exchanger 432 and a gas exchanger 434. In some embodiments, the heat exchanger 432 includes a heat exchanger core 440 including a blood diverter end 442 that is configured to divert blood exiting the blood inlet 416 past hollow fibers 444 through which a heating fluid (e.g., water) can flow. The blood may flow around and past the hollow fibers 444 and thus be suitably heated (or cooled). In some embodiments, the hollow fibers 444 may be polymeric. In some cases, metallic fibers may be used within the heat exchanger 432. According to other embodiments, the heat exchanger 432 includes a metal bellows or other structure comprising a substantial surface area (e.g., fins) for facilitating heat transfer with the blood. In some embodiments, the hollow fibers 444 are hollow polyurethane fibers having an outer diameter between about 0.2 and 1.0 millimeters or, more specifically, between about 0.25 and 0.5 millimeters. The hollow fibers may be woven into mats that can range from about 80 to about 200 millimeters in width. In some embodiments, the mats are in a criss-cross configuration.

In some embodiments, a cylindrical shell 446 may be disposed between the heat exchanger 432 and the gas exchanger 434. In some embodiments, the cylindrical shell 446 may be considered as delineating or defining a boundary between the heat exchanger 432 and the gas exchanger 434. In some embodiments, the cylindrical shell 446 prevents blood from migrating between the heat exchanger 432 and the gas exchanger 434 other than in desired locations. In order to permit blood exiting the heat exchanger 432 to enter the gas exchanger 434, in some embodiments the cylindrical shell 432 includes one or more shell apertures 448.

In the illustrated embodiment, the one or more shell apertures 448 are disposed near an end that is opposite that of the blood inlet 416. As a result, blood entering the heat exchanger 432 from the blood inlet 416 passes through at least a substantial portion of the heat exchanger 432 before the blood can exit the heat exchanger 432 through the one or more shell aperture 448 and into the gas exchanger 434.

In some embodiments the gas exchanger 434 may include a number of microporous hollow fibers 450 through which a gas such as oxygen may flow. The blood may flow around and past the hollow fibers 450. Due to concentration gradients, oxygen may diffuse through the microporous hollow fibers 450 into the blood while carbon dioxide may diffuse into the hollow fibers and out of the blood. In some embodiments, the hollow fibers 450 are made of polypropylene and have an outer diameter of about 0.38 millimeters. According to other embodiments, the microporous hollow fibers have a diameter of between about 0.2 and 1.0 millimeters or, more specifically, between about 0.25 and 0.5 millimeters. The hollow fibers 450 may be woven into mats that can range from about 80 to about 200 millimeters in width. In some embodiments, the mats are in a criss-cross configuration.

In some embodiments, as illustrated, the filter housing 414 defines a filter volume 452 between the filter housing 414 and the apparatus housing 412. A filter assembly 454, including a filter frame 456 and a filter net 458, divides the filter volume 452 into a first chamber 460 that is defined at least in part between the filter assembly 454 and the apparatus housing 412 and a second chamber 462 that is defined at least in part between the filter assembly 454 and the filter housing 414.
The filter frame 456 may be formed of any desired material such as a polymer. In some embodiments, the filter net 458 is a polyester net or a polypropylene net. In some embodiments, at least portions of the filter assembly 454 may be coated with a biocompatible material. In some embodiments, the first purge port 428 is in fluid communication with the first chamber 460 and the second purge port 430 is in fluid communication with the second chamber 462.

In some embodiments, blood passing through the gas exchanger 434 passes through one (not according to the claims) or more (according to the claims) openings 464 into the first chamber 460 of the filter volume 452. At least some of the air bubbles, if any, within the blood may be purged through the first purge port 428. Blood may then pass through the filter net 458 into the second chamber 462 of the filter volume 452. Any air bubbles still within the blood may be purged through the second purge port 430 before the blood exits the blood processing unit 410 through the blood outlet 418.

The plurality of openings 464 are best seen in Figure 11. In some embodiments, as illustrated, the plurality of openings 464 are disposed along an arcuate path. While in Figure 11, the arcuate path is shown as concave relative to the end of the blood processing apparatus 410 at which the blood inlet 416 is located, in some embodiments the openings 464 may instead curve in a convex relation to the end of the blood processing apparatus 410. In some embodiments, the arcuate path may instead take a sinusoidal shape. The one or more openings 464 are sized and positioned to permit a desired volume of blood flow through the blood processing apparatus 410.

The filter assembly 454 is best seen in Figure 12. In some embodiments, the filter frame 456 includes a first annular frame ring 500 having a first diameter and a second annular frame ring 502 having a second diameter that is greater than the first diameter. In some embodiments, as can be seen for example in Figure 10, the filter housing 414 and the filter assembly 454 may both be tapered, but may taper in opposite directions. In some embodiments, tapering the filter assembly 454 can slow blood flow through the filter net 458 and can provide advantages in removing air bubbles from the blood. In some embodiments, an opposite taper in the filter housing 414 may further aid in bubble removal.

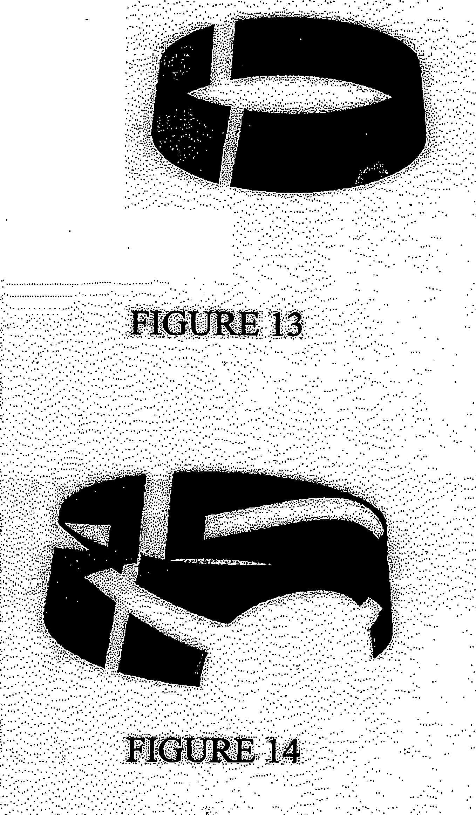

As shown in Figure 12, the filter frame 456 includes one or more bridge elements 504 that extend between the first annular frame ring 500 and the second annular frame ring 502. The filter frame 456 also includes one or more arcuate ribs 506. By comparing Figures 11 and 12, it can be seen that the one or more arcuate ribs 506 are configured to align with the one or more openings disposed within the apparatus housing 412 along the arcuate path 464. As a result, and as will be described with respect to Figures 13 and 14, this alignment can deflect or otherwise impact (e.g., reduce) blood flow velocity through the filter volume 452. In some embodiments, if the path 464 is formed in a different shape than that illustrated, the one or more ribs 506 may be similarly shaped. In some embodiments, the filter frame 456 also includes a plate portion 508 that is arranged near the blood outlet 418 to limit preferential flow in the area near the blood outlet 418.

In some embodiments, the filter frame 456 is configured to regulate blood flow velocity through the filter assembly 454. Figure 13 is a graphical representation of blood flow velocity through a filter lacking the filter frame 456 while Figure 14 provides a graphical representation of blood flow velocity through the filter assembly 452. In both cases, the volumetric blood flow is quite similar, ranging from about 1.61 to about 1.63 liters per minute. These computerized modeling results reveal that the filter frame 456 reduces the maximum blood flow velocity through the filter by as much as 65 percent, without negatively impacting the volumetric blood flow.

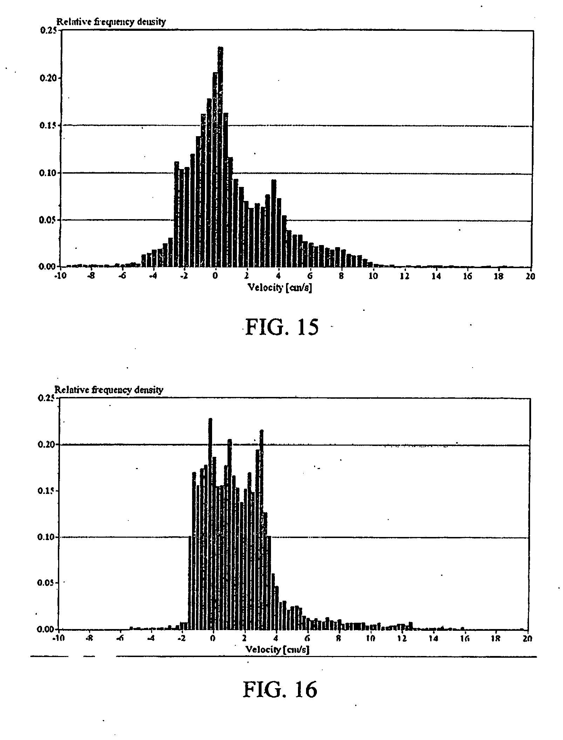

In some embodiments, backflow, or blood flowing backwards through the filter assembly can be problematic. Figures 15 and 16 are relative frequency density graphs, which show that backflow is reduced using the filter frame assembly 454. In these graphs, the relative amount of backflow can be seen by comparing the relative frequency of negative velocities with the relative frequency of positive velocities. Negative velocities represent blood flowing backwards through the filter net 458 while positive velocities represent blood flowing forwards, or in a desired direction, through the filter net 458.

In Figure 15, which represents blood flow without the inventive filter frame assembly 454 and which corresponds to the velocity profile shown in Figure 13, the total amount of backflow is about 51 percent of total blood flow. In Figure 16, which represents blood flow with the inventive filter frame assembly 454 and which corresponds to the velocity profile shown in Figure 14, the total amount of backflow is only about 16 percent.

## Claims

1. A blood processing apparatus comprising:
an apparatus housing (412) having a blood inlet (416) and a blood
outlet (418), the blood inlet (416) extending into an interior of the apparatus housing (412);
a heat exchanger (432) disposed about the blood inlet (416) and in fluid
communication therewith;
a gas exchanger (434) disposed about the heat exchanger (432) and in
fluid communication therewith;
a filter housing (414) coupled about the apparatus housing (412) and
defining a filter volume (452) between the apparatus housing (412) and the filter housing (414), the filter volume in fluid communication with the gas exchanger (434) via a plurality of openings (464) formed within the apparatus housing (412) such that blood exiting the gas exchanger can pass into the filter volume (452); and
a filter assembly (454) disposed within the filter housing (414), the filter
assembly including a filter frame (456) having one or more ribs (506) and a filter net (458) disposed on the filter frame, the one or more ribs (506) aligned with at least a portion of the plurality of openings (464) so as to reduce blood velocity through at least a portion of the filter net (458).

2. The blood processing apparatus of claim 1, wherein the filter frame (456) includes a plate portion (508) arranged near the blood outlet (418) to limit preferential blood flow through the filter assembly (454) near the blood outlet (418).

3. The blood processing apparatus of claim 1, wherein the filter assembly (454) divides the filter volume into a first chamber (460) between the filter assembly (454) and the apparatus housing (412) and a second chamber (462) between the filter assembly (456) and the filter housing (414).

4. The blood processing apparatus of claim 3, further comprising a first purge port (428) in fluid communication with the first chamber (460) and a second purge port (430) in fluid communication with the second chamber (462).

5. The blood processing apparatus of claim 1, wherein the plurality of openings (464) is arranged along an arcuate path, and the one or more ribs (506) comprise arcuate ribs aligned with the arcuate path.

6. The blood processing apparatus of claim 4, wherein the filter housing (414) has a frustoconical configuration having a smaller diameter at one end and a larger diameter at an opposing end, and the second purge port (430) is located near the larger diameter end of the filter housing (414).

7. The blood processing apparatus of claim 6, wherein the first purge port (428) is located near the smaller diameter end of the filter housing (414).

8. The blood processing apparatus of claim 4, wherein bubbles within the blood can exit through the first purge port (428) and/or the second purge port (430).

9. The blood processing apparatus of claim 1, wherein the gas exchanger (434) is configured to permit gas to flow therethrough in order to add oxygen and remove carbon dioxide from the blood passing through the gas exchanger (434).

10. The blood processing apparatus of claim 1, wherein the filter assembly (454) includes a biocompatible coating on the filter net (458).

11. The blood processing apparatus of claim 1, wherein the filter net (458) comprises a polyester filter net or a polypropylene filter net.

## Patentansprüche

1. Blutverarbeitungsgerät, umfassend:
ein Gerätegehäuse (412) mit einem Bluteinlass (416) und einem Blutauslass (418), wobei sich der Bluteinlass (416) in einen Innenraum des Gerätegehäuses (412) erstreckt;
einen Wärmetauscher (432), der um den Bluteinlass (416) vorgesehen ist und in Fluidverbindung mit diesem steht;
einen Gastauscher (434), der um den Wärmetauscher (432) angeordnet ist und in Fluidverbindung mit diesem steht;
ein Filtergehäuse (414), das um das Gerätegehäuse (412) herum angebracht ist und ein Filtervolumen (452) zwischen dem Gerätegehäuse (412) und dem Filtergehäuse (414) definiert, wobei das Filtervolumen durch eine Mehrzahl von Öffnungen (464), die im Gerätegehäuse (412) derart ausgestaltet sind, dass Blut, das den Gastauscher verlässt, in das Filtervolumen (452) passieren kann, mit dem Gastauscher (434) in Fluidverbindung steht; und
eine Filtervorrichtung (454), die innerhalb des Gerätegehäuses (414) angeordnet ist, wobei die Filtervorrichtung einen Filterrahmen (456) mit einer oder mehreren Rippen (506) und ein Filternetz (458), das an dem Filterrahmen angebracht ist, umfasst, wobei die eine oder die mehreren Rippen (506) zu wenigstens einem Teil der Mehrzahl von Öffnungen (464) derart ausgerichtet ist, dass die Blutgeschwindigkeit durch zumindest einen Abschnitt des Filternetzes (458) reduziert wird.

2. Blutverarbeitungsgerät nach Anspruch 1, **dadurch gekennzeichnet, dass** der Filterrahmen (456) einen plattenförmigen Abschnitt (508) umfasst, der benachbart zum Blutauslass (418) angeordnet ist, um einen bevorzugten Blutfluss durch die Filtervorrichtung (454) nahe des Blutauslasses (418) einzugrenzen.

3. Blutverarbeitungsgerät nach Anspruch 1, **dadurch gekennzeichnet, dass** die Filtervorrichtung (454) das Filtervolumen in eine erste Kammer (460) zwischen der Filtervorrichtung (454) und dem Gerätegehäuse (412) und eine zweite Kammer (462) zwischen der Filtervorrichtung (456) und dem Filtergehäuse (414) teilt.

4. Blutverarbeitungsgerät nach Anspruch 3, weiterhin umfassend einen ersten Reinigungsanschluss (428) in Fluidverbindung mit der ersten Kammer (460) und einen zweiten Reinigungsanschluss (430) in Fluidverbindung mit der zweiten Kammer (462).

5. Blutverarbeitungsgerät nach Anspruch 1, **dadurch gekennzeichnet, dass** die Mehrzahl der Öffnungen (464) entlang eines gekrümmten Pfades angeordnet ist, und die eine oder mehrere Rippen (506) gekrümmte Rippen umfassen, die zu dem gekrümmten Pfad ausgerichtet sind.

6. Blutverarbeitungsgerät nach Anspruch 4, **dadurch gekennzeichnet, dass** das Filtergehäuse (414) eine kegelstumpfförmige Gestalt hat, die einen geringeren Durchmesser an einem Ende und einen größeren Durchmesser an einem entgegengesetzten Ende aufweist, und der zweite Reinigungsanschluss (430) nahe von dem Ende des Filtergehäuses (414) mit dem größeren Durchmesser angeordnet ist.

7. Blutverarbeitungsgerät nach Anspruch 6, **dadurch gekennzeichnet, dass** der erste Reinigungsanschluss (428) nahe des Endes des Filtergehäuses (414) mit dem kleineren Durchmesser angeordnet ist.

8. Blutverarbeitungsgerät nach Anspruch 4, **dadurch gekennzeichnet, dass** Bläschen im Blut durch den ersten Reinigungsanschluss (428) und/oder den zweiten Reinigungsanschluss (430) austreten können.

9. Blutverarbeitungsgerät nach Anspruch 1, **dadurch gekennzeichnet, dass** der Gastauscher (434) derart ausgestaltet ist, dass er Gas durchströmen lässt, um dem Blut, das durch den Gastauscher (434) strömt, Sauerstoff hinzuzufügen und Kohlendioxid aus diesem zu entfernen.

10. Blutverarbeitungsgerät nach Anspruch 1, **dadurch gekennzeichnet, dass** die Filtervorrichtung (454) eine biokompatible Beschichtung auf dem Filternetz (458) aufweist.

11. Blutverarbeitungsgerät nach Anspruch 1, **dadurch gekennzeichnet, dass** das Filternetz (458) ein Polyesterfilternetz oder ein Polypropylenfilternetz umfasst.

## Revendications

1. Appareil de traitement du sang comprenant :
un boîtier d'appareil (412) ayant une entrée du sang (416) et une sortie du sang (418), l'entrée du sang (416) s'étendant dans un espace intérieur du boîtier d'appareil (412) ;
un échangeur de chaleur (432) disposé autour de l'entrée du sang (416) et en communication fluidique avec celui-ci ;
un échangeur de gaz (434) disposé autour de l'échangeur de chaleur (432) et en communication fluidique avec celui-ci ;
un boîtier de filtre (414) couplé autour du boitier d'appareil (412) et définissant un volume de filtre (452) entre le boîtier d'appareil (412) et le boîtier de filtre (414), le volume de filtre étant en communication fluidique avec l'échangeur de gaz (434) par l'intermédiaire d'une pluralité d'ouvertures (464) formées à l'intérieur du boîtier d'appareil (412) de sorte que le sang sortant de l'échangeur de gaz puisse passer dans le volume de filtre (452) ; et
un ensemble de filtre (454) disposé à l'intérieur du boîtier de filtre (414), l'ensemble de filtre comportant un cadre de filtre (456) ayant une ou plusieurs nervure(s) (506) et un filet de filtre (458) disposé sur le cadre de filtre, la ou les plusieurs nervure(s) (506) étant alignée(s) avec au moins une partie de la pluralité d'ouvertures (464) de manière à réduire la vitesse du sang à travers au moins une partie du filet de filtre (458).

2. Appareil de traitement du sang de la revendication 1, dans lequel le cadre de filtre (456) comporte une partie de plaque (508) agencée à proximité de la sortie du sang (418) pour limiter le débit sanguin préférentiel à travers l'ensemble de filtre (454) à proximité de la sortie du sang (418).

3. Appareil de traitement du sang de la revendication 1, dans lequel l'ensemble de filtre (454) divise le volume de filtre en une première chambre (460) entre l'ensemble de filtre (454) et le boîtier d'appareil (412) et une deuxième chambre (462) entre l'ensemble de filtre (456) et le boîtier de filtre (414).

4. Appareil de traitement du sang de la revendication 3, comprenant en outre un premier orifice de purge (428) en communication fluidique avec la première chambre (460) et un deuxième orifice de purge (430) en communication fluidique avec la deuxième chambre (462).

5. Appareil de traitement du sang de la revendication 1, dans lequel la pluralité d'ouvertures (464) est agencée le long d'un trajet arqué, et la ou les plusieurs nervure(s) (506) comprend/comprennent des nervures arquées alignées avec le trajet arqué.

6. Appareil de traitement du sang de la revendication 4, dans lequel le boîtier de filtre (414) présente une configuration tronconique ayant un petit diamètre au niveau d'une extrémité et un grand diamètre au niveau d'une extrémité opposée, et le deuxième orifice de purge (430) est situé à proximité de l'extrémité à grand diamètre du boîtier de filtre (414).

7. Appareil de traitement du sang de la revendication 6, dans lequel le premier orifice de purge (428) est situé à proximité de l'extrémité à petit diamètre du boîtier de filtre (414).

8. Appareil de traitement du sang de la revendication 4, dans lequel des bulles dans le sang peuvent sortir à travers le premier orifice de purge (428) et/ou le deuxième orifice de purge (430).

9. Appareil de traitement du sang de la revendication 1, dans lequel l'échangeur de gaz (434) est configuré pour permettre au gaz de s'écouler à travers celui-ci afin d'ajouter de l'oxygène et d'éliminer le dioxyde de carbone du sang passant à travers l'échangeur de gaz (434).

10. Appareil de traitement du sang de la revendication 1, dans lequel l'ensemble de filtre (454) comporte un revêtement biocompatible sur le filet de filtre (458).

11. Appareil de traitement du sang de la revendication 1, dans lequel le filet de filtre (458) comprend un filet de filtre en polyester ou un filet de filtre en polypropylène.
